# EUROPEAN PATENT APPLICATION

(11) **EP 1 288 835 A1**
(43) Date of publication of application: **05.03.2003**
(21) Application number: 01650098.5
(22) Date of filing: 31.08.2001
(51) Int. Cl.: G06F 19/00, C12Q 1/68

(54) **A method for defining gene hunting studies**

(71) Applicant: Hitachi, Ltd., Chiyoda-ku, Tokyo 101-8010 (JP)
(72) Inventor: Sievers, Fabian, Sandymount, Dublin 4 (IE)
(74) Representative: O'Connor, Donal Henry

(57) **Abstract**

The invention relates to a method for defining or controlling a gene hunting study which is used to determine certain genetic data. Essentially, what is done is to specify the loci to have an initial population specifying the loci of reference markers and the locus of at least one test locus which corresponds to what is postulated to be the locus where a mutation may be causing a particular disease. The recombination fraction is obtained between all the loci and with this initial data, a test population of individuals is generated. This then has a theoretical patient population having the mutation. This theoretical patient population can then be used to allow an initial assessment of the gene hunting studies. For example, the theoretical patient population can be used to carry out the gene hunting study and the results of the gene hunting study on the theoretical population can then be used to specify and define actual gene hunting studies. The invention can also be used to test the efficacy of gene hunting studies.

## Description

### Introduction

The present invention relates to a method and system for defining gene hunting studies.

Gene hunting studies are used to determine certain genetic data. A crucial first step in finding diseased loci that contribute to a genetic disease is to demonstrate linkage with a gene or DNA sequence of known location, namely, a marker. Various methods have been used such as population association between disease and a marker. However, the problem with inferring linkage by population association is that association can occur in the absence of linkage, for example, as a result of population stratification. Thus, tests that do not depend on association have been carried out by, for example, family-based controls tests or transmlssion/disequilibrium tests.

However, irrespective of the gene hunting study, be it association or family-based, it requires an initial design of experiment. For example, how big a stretch of the genome should be spanned, what density of marker should be used, which kind of markers and most importantly, how many patients or families. Then, one has to ask questions as to whether the total population is big enough to support a meaningful analysis or does one have to move to a different bigger population.

There is a further problem in that when a geneticist has information regarding a number of patients, the geneticist has to form a hypothesis based on analysis of the genetic data of these patients and then it is necessary to check whether it is a sound hypothesis or not. It is only at that stage, after the hypothesis has been checked, whether one can state with any certainty whether the gene hunting study is useful or not. This means, in effect, that the geneticist has to do all the work before having any idea as to whether the gene hunting study has been correctly designed or not. Ideally, any algorithm should map unambiguously to a particular program. One way to assess that an algorithm has been designed carefully and encoded correctly is to test it with data that is known to exhibit linkage or not.

Experimental data in itself is generally not sufficient since we do not know where linkage occurs and where it does not. Calibration with existing implementations and alternative algorithms has the drawback that we have to confident that these produce the correct answer.

There are therefore two major problems. The first major problem is to ensure that the particular analysis methodology which will generally be analysis software, has been correctly designed so that when used on the data, it should produce the desired result. Secondly, one has to then be assured that the actual experiment design itself is correct. The two essentially go hand in hand in the sense that if the analysis software is flawed, then it does not matter how well the experiment is being designed and carried out as the results will be flawed. Similarly, even if the analysis software is correct and well designed, if the actual study itself has been badly designed, then the experiments will fail. The gene hunting study therefore depends on both of these.

The present invention is directed towards solving these problems.

### Statements of Invention

According to the invention, there is provided a method of defining a gene hunting study to determine certain genetic data comprising:-
specifying the loci of reference markers;
specifying the locus of at least one test locus;
obtaining the recombination fraction between all the loci;
defining an initial population including at least its size and allelic data for the generation of a test population of individuals identified by their alleles;
generating a test population from the initial population by causing the initial population to grow into a test population;
inputting a mutation at the or each test locus during the generation of the test population; and
selecting the required genetic data from the test data having regard to the mutation and reference markers to provide a theoretical patient population having the mutation to allow an initial assessment of the gene hunting study.

Subsequently, the steps may be carried out of:-
using the theoretical patient population to carry out a gene hunting study; and
using the results of this gene hunting study to specify an actual gene hunting study.

The advantage of this is that one can carry out the gene hunting study theoretically to decide whether such a gene hunting study is likely to be successful. If carried out in practice, it is possible that having taken some of the theoretical patient population to carry out the gene hunting study, it would quickly be apparent that such a gene hunting study would not lead to a result that could be relied on and therefore a larger part of the theoretical patient population would then be used to carry out the gene hunting study of the invention again until a satisfactory result was achieved. Then, it would be possible for the researcher to define and design the practical gene hunting study with a measure of confidence or to decide it was not practical to carry it out.

In one method according to the invention, the generation of the test population comprises:-
selecting parents from the initial population in accordance with predetermined parental choice rules;
determining the number of offspring for each set of parents in accordance with predetermined birth rate rules;
assigning a gender to each offspring in accordance with predetermined gender rules;
allocating genes to each offspring allowing for random recombinations in accordance with specified recombination parameters; and
retiring members of the population in accordance with predetermined mortality rates.

It will be appreciated that this allows a test population to be provided which will mirror the real population.

The recombination fraction assigned to the test locus is obtained by taking a portion of the recombination fraction of the two adjacent markers. In one embodiment, the recombination fraction assigned is half the recombination fraction of the two adjacent markers. It is also possible to assign more than one test locus between adjacent markers and when this is done, the sum of the recombination fractions is a proportion of the recombination fraction between the markers and cannot be more than the latter fraction.

Ideally, the test population is chosen after many generations of evolution.

When the test population is being generated, at predetermined numbers of generations, a correlation between reference alleles and other alleles is determined and when the correlation is determined to be satisfactory, the test population is chosen to be that size for extraction of a patient size for the subsequent study.

The size of the initial population may be varied and the test population determined for different sizes of initial population. The mutation may be introduced in the initial population or many generations beyond the initial population or indeed, at a number of loci.

The size of the theoretical population is varied to determine the size of a subsequent real patent population for the actual gene hunting study.

When the test population provides a theoretical patient population whose size relative to the test population is less than the relative size of the actual patient population the mutation is introduced at at least one additional locus and a new theoretical patient population produced.

Alternatively, when the test population provides a theoretical patient population whose size relative to the test population is greater than the relative size of the actual patient population the size of the initial population is increased and a new test population produced.

It is envisaged that a plurality of theoretical patient populations of different allelic data are chosen for the one gene hunting study and a theoretical gene hunting study is carried out on each theoretical patient population to provide one or more real patient populations for the subsequent actual study.

Ideally, in the method, the efficacy of the gene hunting study in the theoretical patient population is assessed by using a gene hunting study testing methodology.

Indeed, more than one gene hunting study testing methodology may be used as indeed more than one gene hunting study methodology.

Further, the invention provides a method in which an actual gene hunting study including the specification of the loci of reference alleles and size of patient population is defined when a gene study on a theoretical patient population provides an apparently successful study.

Further, the method comprises:-
providing at least some of the data necessary to carry out the method of any preceding claim;
sending the data to a third party;
receiving the results of the method carried out by the third party; and
using the results to carry out a subsequent gene hunting study.

It is envisaged that the invention may provide theoretical gene hunting studies produced by the method as well as an actual gene hunting study which has been defined by the method.

Further, the invention provides a system for control of a gene hunting study to determine certain genetic data including a server comprising:
means to generate a test population from an initial population of a certain size in which the test population is expressed in terms of its allelic data;
means to input a mutation at least at one locus during generation of the test population; and
means to extract a theoretical patient population from the test population having regard to its allelic data.

The system may also comprise means to carry out a gene hunting study on at least part of the theoretical patient population and additionally may comprise means to carry out a study testing methodology.

Further, the invention is envisaged being provided on a computer and ideally the invention will be embodied in a computer program comprising program instructions for causing a computer to run the method as laid out above or indeed, to perform the means for the system as laid out above. Such a computer program may be embodied on a record medium, a computer memory, a read only memory or carried on an electrical signal carrier.

### Detailed Description of the Invention

The invention will be more clearly understood from the following description of some embodiments thereof, given by way of example only, with reference to the accompanying drawings, in which:-
Fig. 1 is a layout of the methods carried out in accordance with the invention,
Fig. 2 is a layout of hardware requirements of the invention,
Fig. 3 is an illustration of a simple pedigree of a population generation,
Fig. 4 is a simple flowchart of the method according to the invention showing procreation with recombination,
Fig. 5 shows loci, alleles and recombination ratios,
Fig. 6 is a diagrammatic overview of the invention,
Fig. 7 is a graphical representation of the results of a test population,
Figs. 8(a) to (c) show the loci at three different generations extracted from Fig. 7, and
Fig. 9 shows the incidence at one locus for all the generations extracted from Fig. 7,
Figs. 10(a) and (b) illustrate the method which was carried out; and
Fig. 11 is an overview similar to Fig. 6 of the results of a gene hunting study.

Essentially, the invention is a relatively simple one. What is done is to prepare an artificial population, using a population generator such as a suitably programmed computer, for example, one could generate four million individuals, forty thousand of whom have a particular genetic disorder, beginning maybe 1,000 years ago with an initial population of 1,000 where one or more of the individuals in that 1,000 initial population would have this particular genetic disorder. The impact of the initial population is that it determines the percentage of affected individuals in the final population in which the patient population is embedded. Thus, what has been done is to define an initial population for generation of a test population, which test population will in turn provide a patient population of affected individuals. That patient population can then be used to determine whether a gene hunting study, for example, using 100 of that patient population, would be sufficient or not. This can then be increased until you find the number of patients where the results would be reliable. Having obtained this artificial population, this in turn will generate artificial data, that is to say, this patient population.

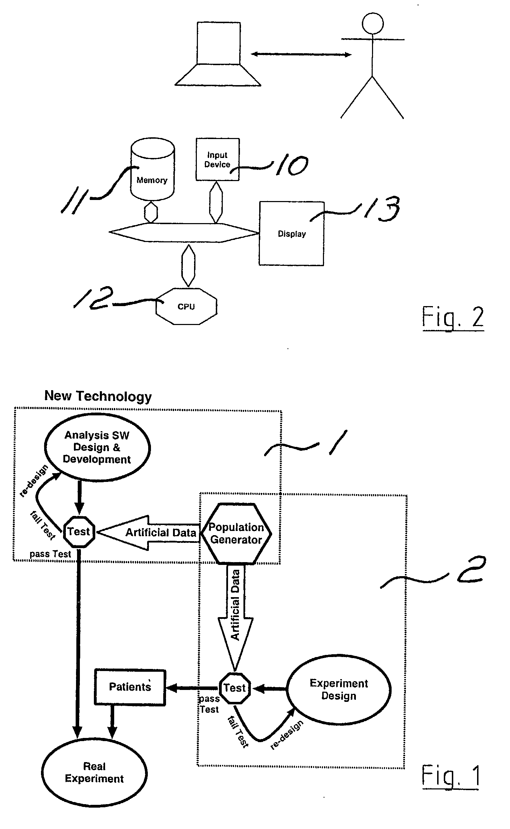

Referring to Fig. 1, the population generator can be used in two ways. Firstly, the artificial data can be sent to a test to analyse the software design and development and until that software for analysing the data has been shown to be accurate, the particular test methodology is not used. This is identified by the area surrounded by the interrupted lines and identified by the reference numeral 1. Once we are satisfied that the particular methodology is a suitable methodology, then it can be used in the experiments. Very often, it will not be necessary check such methodology because it will have already been checked both practically and theoretically. However, it can be said that this particular testing of the algorithms and analysis process is useful as a backup to the experiments.

However, the main purpose of the population generator is illustrated in the portion of the drawing of Fig. 1, identified by the reference numeral 2. A particular experiment design is chosen and the artificial data generated by the population generator is used to test the experiment design and all aspects of the experiment can be altered using the artificial data and in each case testing it. This is a much cheaper way of testing the design which can be done iteratively until it is decided that the particular experiment design, i.e. the definition of the gene hunting study, would lead to useful results in which case the necessary number of real patients is chosen and the real gene hunting study takes place on them. Essentially, therefore, the purpose of the invention is to produce genetic information that is correlated with the particular reference points quickly and accurately.

Referring now to Fig. 2, there is illustrated, in simple form, the hardware requirements for carrying out the invention which comprises an input device 10, a memory 11, a CPU 12 and a display 13. Any suitable computer can be used. When the user submits the relevant parameters to the input device, these are stored in memory, the CPU performs the operation prescribed and the output device delivers the result. Effectively what is being carried out is a process which starts off by developing or defining an initial population for generation of the test population from which the patients are extracted.

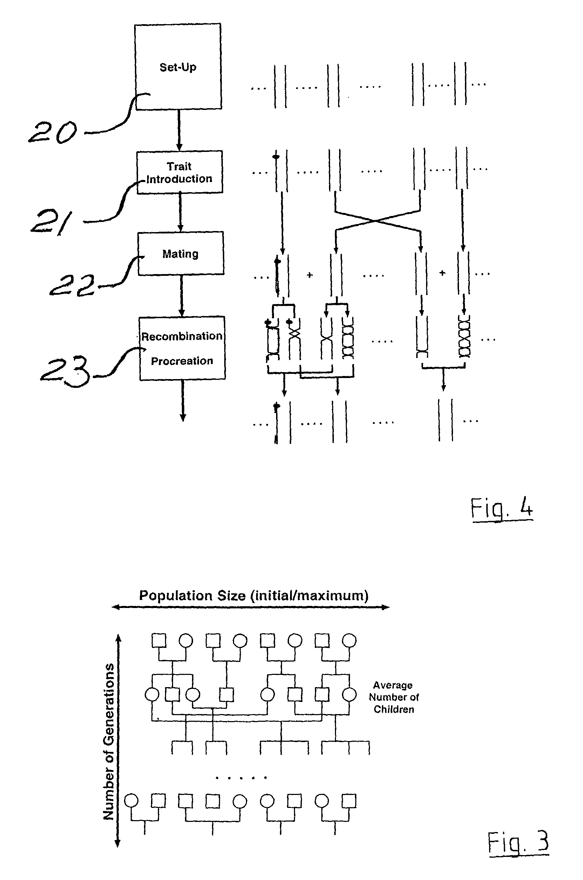

Fig. 3 shows how an initial population, after a number of generations, will provide a further population.

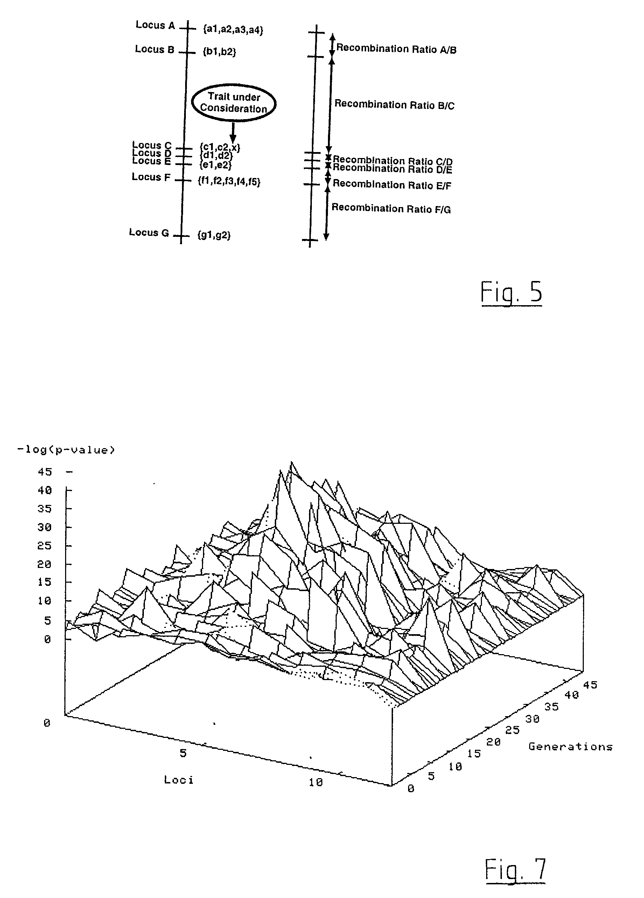

Fig. 4 shows how the genetic material is passed from parent to offspring and so on. All of this is well known to the geneticist and is included for information for those without such knowledge. Every individual, as is known, has two complete sets of alleles but passes on only one. The set that is passed on by one parent is usually not identical with either of the two original sets of this parent but is a mixture of both sets. The probability of two adjacent alleles, that is to say, two alleles of neighbouring loci within the same set, being transmitted together is given by the recombination fraction between the two loci. The recombination ratios have to be identified and are essential. This is visualised in Fig. 4. Thus, dealing simply with the transfer of a trait, then one starts off in step 20, with the set-up of the initial population. In step 21, the trait is introduced. In step 22, mating takes place and in step 23, we have procreation with recombination. Loci, alleles and recombinant ratios are illustrated in Fig. 5.

To generate the population, the first step is to define an initial population and to specify its characteristics. Then, it is necessary to specify the loci of reference markers. Then, the locus of at least one test locus is specified. This is obviously a speculative locus. This test locus is the locus where the disease locus will exist. These are therefore the specified loci, that is to say, the test locus and the loci of the markers. It will be appreciated that there could be any number of markers and similarly, there can be any number of test loci. Then, it is necessary to obtain the recombination fraction between all the specified loci, that is to say, of both the markers and the test locus or loci. This can be obtained for the markers without any difficulty, however, for the test locus, various assumptions have to be made and the recombination fraction for the test locus has to be derived from the recombination fraction of the two adjacent markers. For example, if the recombination fraction between two adjacent markers was 0.3, then if it was decided that the test locus was midway between the two markers, it could be assigned a recombination fraction of 0.15. Alternatively, if it was decided that it was closer to one of the markers, then its recombination fraction with that marker might be smaller and would be larger with the other marker. When there is more than one test locus between the two markers, it will be necessary to ensure that the recombination fraction of the two test loci is not greater than the recombination fraction between the two markers. However, it will be appreciated that this can be easily set up by the experimenter. Thus, for the generation of the test population from which the patient population will be taken, there is defined an initial population including at least its size and allelic data. Then, it is necessary to generate the subsequent population. Thus, parents have to be selected according to certain rules. These rules have to be specified by the experimenter and could be deterministic in nature corresponding to the concept of arranged marriages, but could be selected randomly with special provisions made to restrict consanguinity, intergeneration mating and to remove individuals who are unable to mate because they are too old or indeed deceased. Individuals could engage in more than one pairing but again, to decide on these details, it is up to the experimenter. In general, one would try to mirror mating habits of the initial population as near as possible to the mating habits that were likely to have been the norm in the particular area from which the experiments are to take place. Thus, you would select the parents in accordance with predetermined parental choice rules. You would determine the number of offspring for each set of parents in accordance with predetermined birth rate rules. You must then assign a gender to each offspring in accordance with predetermined gender rules and when all these is done, then you allocate genes to each offspring allowing for random recombinations in accordance with specified recombination parameters. You finally then end up with a population of the offspring, their parents and possibly grandparents. Again, retirement/mortality rules are necessary. You then determine the required genetic data from this test population. That test population allows you to provide a theoretical patient population and the patient population can be used to determine the efficacy of the study. The invention generates an initial population with a certain distribution of alleles. From this, couples are drawn at random, they procreate, couples are drawn again and so on. At any point of one specific simulation, there are only three generations present as a test population, namely, grandparents, parents and children. Before a new generation is produced, the older generation retires. Again, the gender of a child is determined randomly. The alleles are derived from the parents' set of alleles, taking cross-over into consideration. Sex chromosomes may also be taken into account.

As explained above, in the first generation, a mutation is introduced at one locus. To ensure that this mutation, that is to say, this disease gene, does not die out at the early stages of the simulation, this mutation can be introduced into more than one person. All those persons carrying the mutations are clones - they have exactly the same set-up. It is envisaged that it would be possible to change this so that populations could be set-up with more than one person having the mutation but with different sets of alleles. It will be appreciated that the bigger the initial population size, the better the experimental allele frequencies will match the ones in the actual population genetics files. At the end of the evolution, the allele frequencies should be essentially the same as the initially specified frequencies. The final output will contain all the patients within that test population which will have this mutation.

It will be appreciated that the population generation can be modified in many ways. For example, population stratification is allowed after the mutation has been introduced in the first iteration. However, stratification could also be provided before the mutation is introduced. Thus, it would be possible to start with an initial population and then after a certain number of generations had been formed, introduce the mutation.

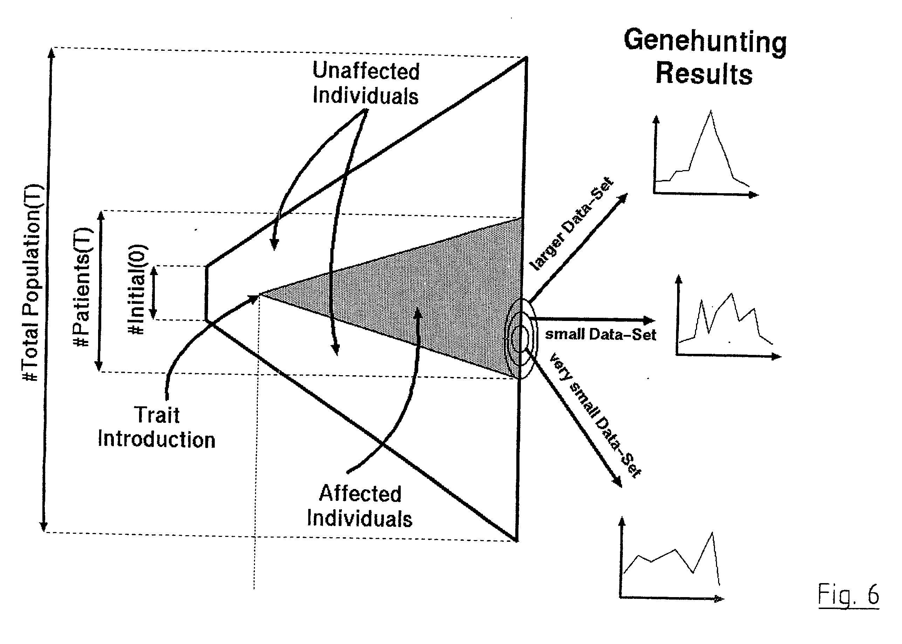

Referring to Fig. 6, the patient and population generation is shown having been produced over a large number of generations. It started off with an initial population and some way through the generation of the population, the trait was introduced.

Then, there were various affected individuals so that you ended up with a test population containing a large patient population. All of this patient population would have a gene at the particular test locus. Then it is a question in gene hunting of deciding how to assess the results and these are shown for a very small data set, there does not appear to be any result that would appear to show any real results, while for a small data set, one can see that there is some result. Finally, for a larger data set of patients, there is a quite definite result. The actual meaning of the results and the parameters are described later.

Putting the invention practically, assume, for example, that there are four million people in a country of whom 40,000 have a particular genetic disorder. There may be only an awareness that 5,000 of these individuals have the disorder and indeed, a further number, for example, only 254, have been genotyped. The question the present invention tries to answer or at least assist in answering, is - is a patient population of 254 individuals enough? To do this, we effectively generate four million individuals, 40,000 of whom have the particular genetic disorder, beginning maybe 1,000 years ago with an initial population of 1,000. The impact of the initial population is that it determines the percentage of affected individuals in the final population in which the patient population is embedded. This final or test population is all important. Thus, the initial population is the input parameter than can adjust the ratio of affected individuals so that you can end up with these forty thousand people having this particular genetic disorder. Having got this 40,000 affected, you would start off by choosing 100, i.e. the very small data set of Fig. 6, affected and conduct the actual gene hunting study theoretically and then possibly find that 100 is not enough and then you would try 200 and still find that this was not enough: this is the small data set of Fig. 6. Then, you would keep on increasing this patient population to a level where your results become reliable. Assuming it is less than the 254 patients who have been genotyped, you can move immediately to the actual gene hunting study. If it is more, you may require further patients to be genotyped, i.e. to get the larger data set of Fig. 6.

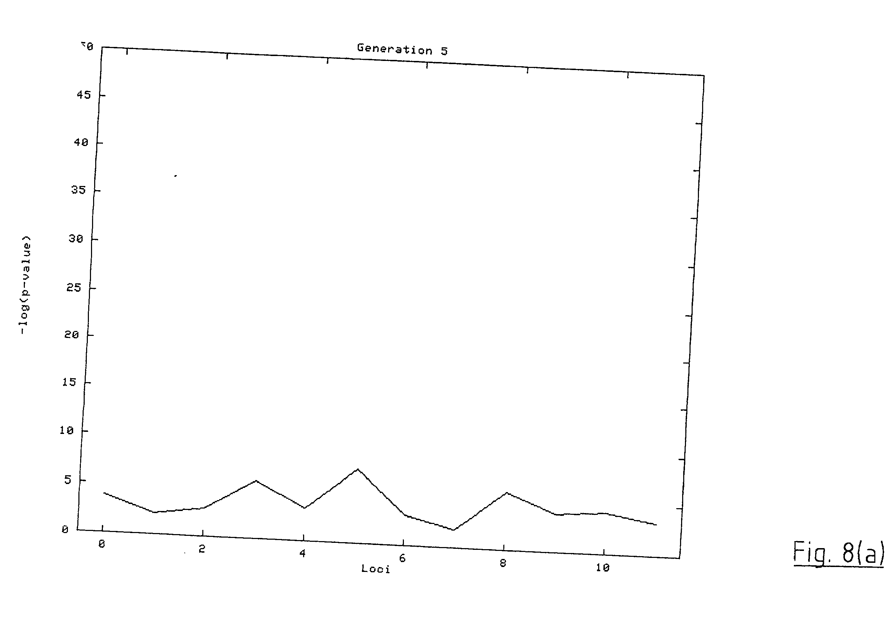

Fig. 7 illustrates the use of the transmission disequilibrium test (TDT) to determine the usefulness of the present invention, which considers parents who are heterozygous for an allele associated with the disease and evaluates the frequency with which that allele or its alternative is transmitted to affected offsprings. Compared with conventional tests for linkage, the TDT has the advantage that it does not require data either on multiple affected family members or on unaffected siblings. This is a well known test procedure.

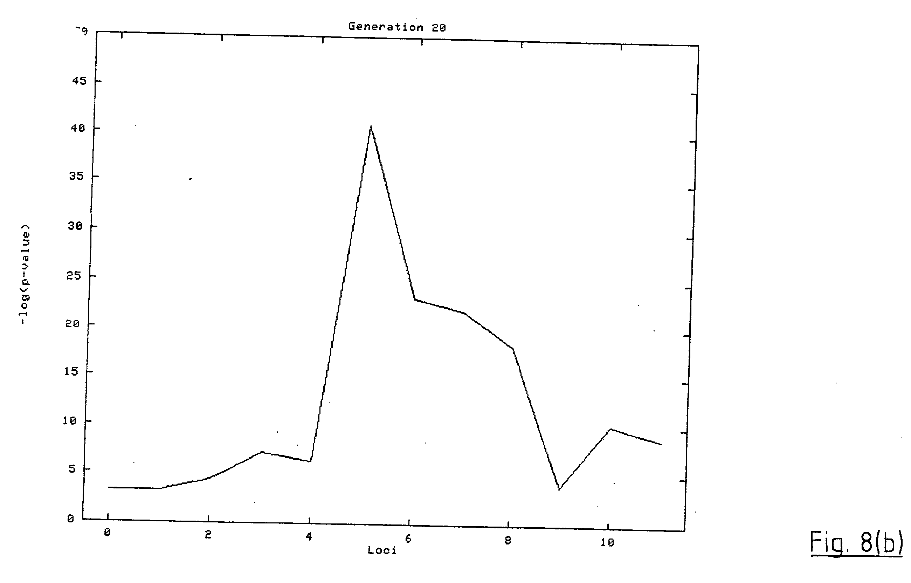

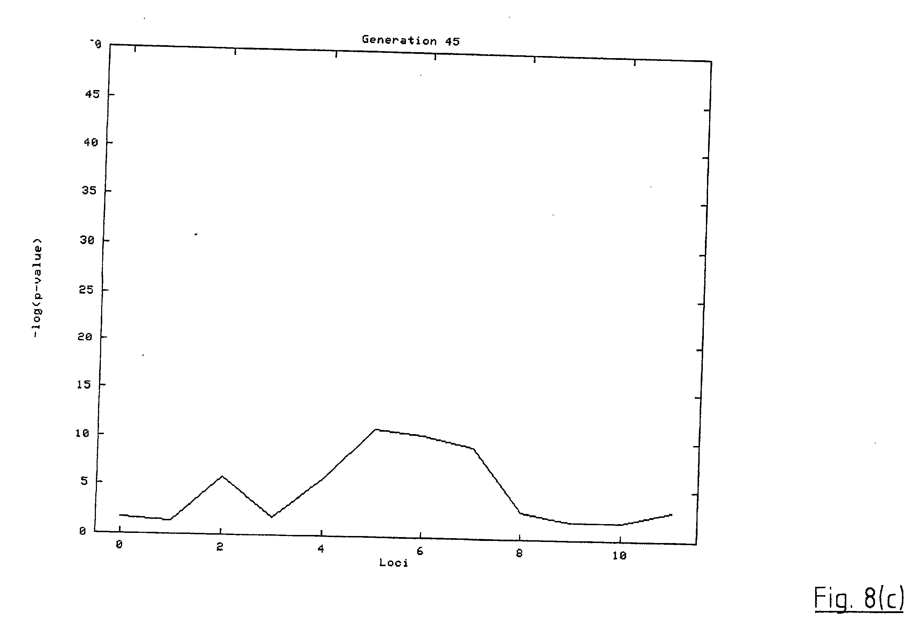

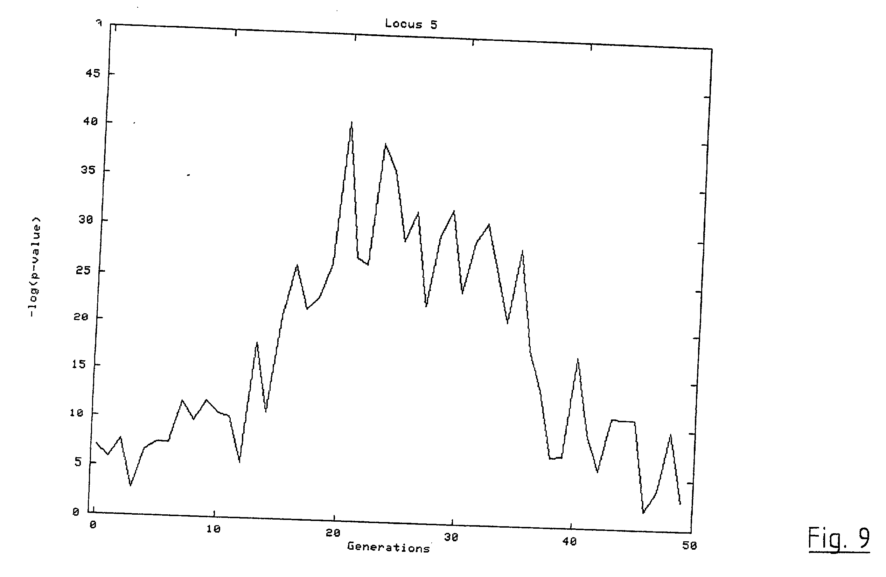

Fig. 9 charts the test population for various generations at one locus, namely locus 5, while Figs. 8(a), 8(b) and 8(c) show the frequency of occurrence at the various loci for different numbers of generation. It can be seen clearly from Fig. 9 that there is a clear level of linkage. You then have a phase of increasing linkage and a phase of decay.

When conducting a gene-hunting study one sometimes has a choice of different methodologies, each of which may have its advantages and drawbacks. If one is interested in establishing linkage of a marker allele to an (unknown) disease one could use, for example, a likelihood-based linkage test, an affected sib-pair test (ASP) (sib, i.e. sibling), a case-control study, the transmission disequilibrium test (TDT) or its extension the pedigree disequilibrium test (PDT), to name just some. One advantage of the case-control study is that for each affected person only two individuals have to be genotyped, that is the affected person and a control case. For the TDT at least three individuals have to be genotyped, that is the affected person and the two parents. Another disadvantage of the TDT is that for late-onset diseases (e.g., Alzheimer) the parents are usually no longer available for genotyping. An advantage of the TDT is that it usually has more 'power' than a simple likelihood-based linkage test.

Most gene-hunting studies involve at some stage a counting and comparison procedure. In likelihood based linkage test we might compare the number of actually observed alleles to the number we would expect under the assumption of no linkage, or in the TDT we compare the number of alleles that are not transmitted from the parents to the affected child to the number of alleles that are transmitted to the affected child. In a study (with real or with simulated patients) we have to genotype the patients. For simulated patients this is simple as the alleles at the marker loci are readily legible. For real patients tissue samples have to be taken, and a sequencing machine determines the alleles at each marker locus. For real patients the genetic phase is usually not known, that is, we do not know which allele is of paternal and which is of maternal origin, and one has to go to great trouble and expense to obtain this information. For simulated data this information is readily available. Some algorithms that can make use of the gametic phase will run orders of magnitude faster than in the phase unknown case.

If it is decided to use the TDT to establish linkage we have to use a particular implementation of this algorithm. In principle, the analysis could be carried out manually for this particular algorithm, but other algorithms are so computationally intensive that using a computer is essential. For the TDT several implementations are available, one example is the TDT facility of the Genehunter software package provided by the Rockefeller Center. This software package like many others can be obtained freely and will execute on whatever machine it is installed on. The input data has to be arranged in the required input format. For Genehunter this would include a personal identifier, the parents' personal identifier, gender information, affectation status and (phase unknown) allelic data. The program is executed and provides output that lists the numbers of un/transmitted alleles, the corresponding chi-squared value and the so called p-value that is obtained from the chi-squared value. The p-value is the probability that states how likely it would have been for this particular allele at this particular locus to have occurred in the observed way purely at random. Low p-values suggest that pure chance cannot explain the observed distribution of alleles and one might be led to believe that an underlying phenomenon could have contributed to this effect. One would hope that this underlying phenomenon is linkage.

In the results that we received for a population of theoretical patients where we introduced a disease locus at a well defined position we modified the output from the Genehunter software package. Firstly, as the p-values of alleles at loci close to the introduced disease locus were very small we did not plot the p-value itself but its negative logarithm. Secondly, as we were mainly interested in pin- pointing the disease locus we only plotted the result for one allele per locus. Genehunter will produce one p-value for every allele at every locus. We selected the allele with the smallest p-value (greatest negative logarithm) for each locus. This would not be very useful for a real gene-hunting study where one would like to establish linkage between the disease and a particular allele at a particular locus but it served to assess the efficacy of the experiment design, as shown below.

An initial population was set up whose alleles were chosen at random. The probability distributions according to which the alleles would be selected by the computer would be the same as the distributions of alleles observed in nature. We found empirically that the distribution of alleles changes only very little during the evolution, so that the final allele distribution of the theoretical population can be made to match the distribution of alleles observed in the real patient population. One disease carrying patient was inserted into the initial population. The ratio of the size of the initial population and the number of inserted disease carriers has a bearing on the ratio of simulated disease carriers, i.e. the theoretical patient population, at the end of the simulation and can therefor be chosen to match the observed ratio of disease carriers in the (real) population. The test population evolved over a certain number of generations. This number can be fixed by the experimenter if the age of the disease can be surmised. If no estimation can be made one could generate a couple of generations, some after a small number of generations and some after a larger number of generations. It is up to the experimenter to decide what 'small' and 'large' means in this context but the present invention can assist in this decision making process.

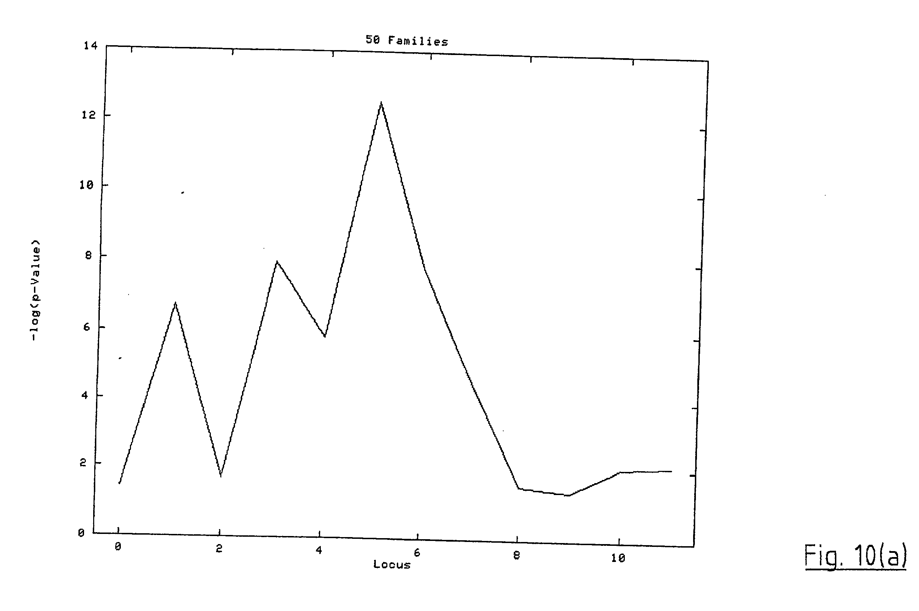

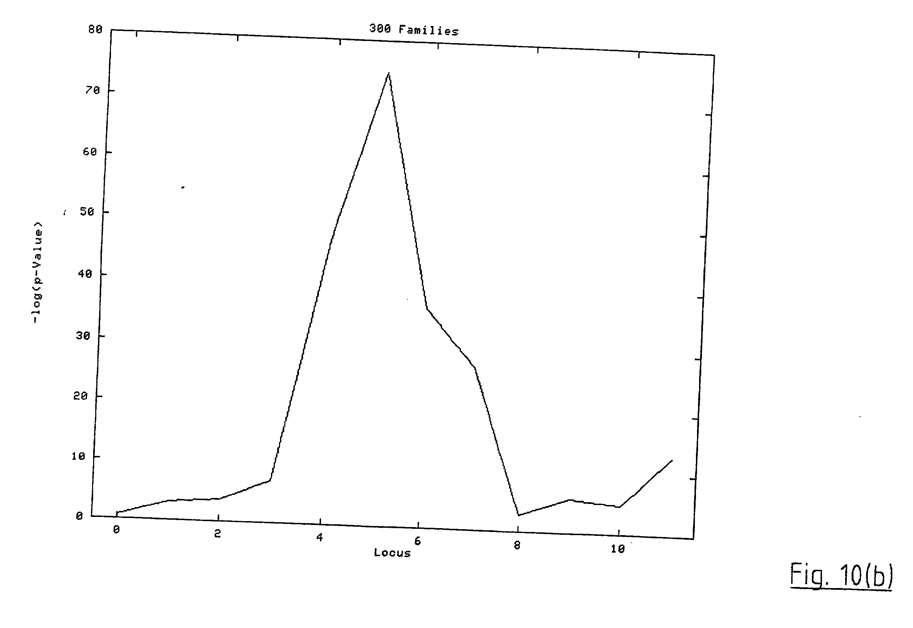

In one method, a test population was provided a certain size containing a sub-set of individuals that carried the disease allele which had been introduced into the initial population. This sub-set we call the theoretical patient population. At this point the actual experiment design phase commences. We decided to use the TDT because we anticipated having access to the affecteds' parents. The second step in designing the experiment was to select the size of the real patient population that would be genotyped. Genotyping is expensive and experimenters would therefore like to keep this number as small as possible, while still giving reliable answers. Out of the theoretical patient population we selected a small sub-set of families and used the Genehunter software. The condensed results (only the allele with the maximum of -log(p-value) at a locus) is represented in Figs. 10(a) and 10(b).

Referring to Fig. 10(a), for a first test which was carried out on fifty families, it is clear that there is a peak at locus 5, however, the distribution as a whole is very broad and it will be noted, it exhibits additional peaks. It was decided that probably the size of the patient population was too small and a new bigger subset of the theoretical patient population, namely, a population of 300 families was chosen.

This time the inspection of the results produced by the Genehunter package were satisfactory. The peek appeared in the immediate vicinity of the locus where we had introduced a disease causing reference allele, the distribution as a whole was very pronounced and no other loci seemed to exhibit linkage of a comparable magnitude than the location of the peek. This can be seen in Fig. 10(b).

We would therefor assume that a real gene-hunting study, that would use the same markers as specified in our simulation and would employ the TDT should draw on a data-set of genotyped patients that is significantly larger than our first, small sub- set and should be comprised of as many patients as we had used in our second, larger data-set to be able to locate a real disease gene at the locus where we had introduced a reference allele. This result does not apply for studies that expect to find a disease locus at a different locus other than the one introduced by us. To be able to find a disease locus in a wide stretch the above procedure will have to be repeated and reference alleles will have to be introduced at different loci.

It is not expected that sensitivity studies for simple diseases (caused by one disease gene at one locus) would directly apply to complex diseases, where the disease is caused by the interaction of various genes at different loci. In this case we have to introduce several reference alleles at different loci into our theoretical initial population and follow through the steps described above.

What must be appreciated is that to date the experimental data has not been fully checked out with known gene hunting studies, however, it is expected that such will be done over a period of time. It is also possible that when practical results are obtained of gene hunting studies and are compared with the theoretical gene hunting studies and the results obtained therein, the method according to the present invention will be changed in various ways. One of the obvious matters that needs to be investigated further is whether any particular algorithm accurately detects the location of the disease gene which had been introduced at the test locus or loci. Essentially, the only way to accurately test any particular algorithm is to test it in a very large data set which in this context would be the maximum number of real patients we could ever genotype, that is, the number of all real patients in a genetically closed group that suffer from a disease which it was required to localize. An example for a genetically closed group could be the inhabitants of Iceland, the Amish or others. Obviously, other more powerful algorithms will be developed over time.

It is also important to again emphasise that the generation of a theoretical patient population is a random experiment and that the theoretical patient population obtained is therefore just one possible population that provides only an estimate of a real patient population and particularly a real population's characteristics. Thus, it is expected that in practice, many operations of the method may have to be carried out varying the parameters slightly.

In another way of carrying out the invention, the researcher inserts a large number of test loci. For example, in this particular experiment, three test loci were inserted between each pair of marker loci. In the particular example, one is placed close to one marker, the other is placed close to the other marker and a third test locus is placed between both markers. In this particular experiment, special provisions were made for the insertion of reference loci at the ends. These test loci would have only two types of alleles, namely, trait and non-trait.

The trait alleles can be inserted into one individual or into many, they could be inserted at one generation or over many generations, but all reference loci will be filled before the simulation is over.

After T generations we have generated a 'theoretical patient population' with many (may-be all) individuals carrying one or many reference alleles at the reference loci between the marker loci.

The experimenter can then carry out his gene-hunting study assuming there is only one disease causing gene at reference locus A. This is done by picking out only patients that carry a trait at locus A and ignoring all patients that have no trait allele at locus A, despite having trait alleles at loci B and C.

The experimenter can then assume that the disease is caused by a single trait gene at reference locus B, ignoring trait genes at loci A and C. For this the 'theoretical patient population' does not have to be re-generated, only a different reference locus is projected out. This can be repeated for all assumed single locus disease loci.

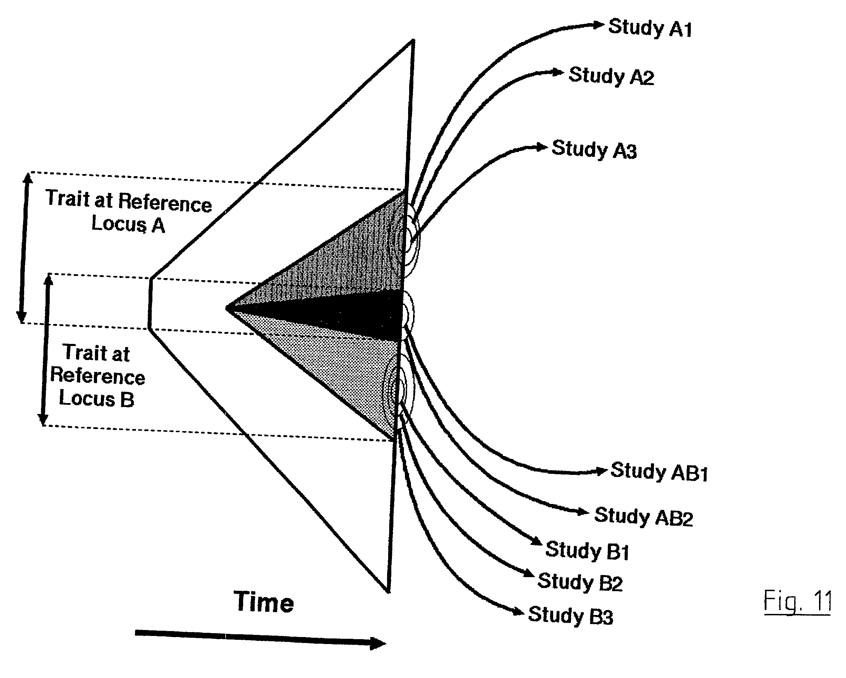

Referring to Fig. 11, the experimenter could then look at complex diseases that are caused if an individual has trait alleles at reference loci A and B, omitting individuals that have no trait alleles at locus A and locus B or individuals that have trait alleles at locus A but not at locus B and vice versa, etc.

More complex scenarios involving large numbers of contributing trait alleles at designated test loci are envisaged.

It is envisaged that the insertion of reference or test loci can be automated, and that the reference loci are picked for gene-hunting by the experimenter after the test population has been generated. The advantage is that a population will have to be generated only once. A disadvantage would be that the execution time will be longer than for a single reference locus simulation. The execution time will be less than several simulations for single reference loci.

It will be appreciated that there are many ways in which the invention can be carried out. For example, in generating the test population, various initial populations can be used in accordance with the desires of the experimenter. For example, the initial population could be devised having regard to what the experimenter wished to achieve in the final population in relation to the test population. Needless to say, various rules for determining the number of offspring, the gender of each offspring and indeed, how members of the population can be retired, can all be provided by the experimenter, either from data already available or simply randomly.

It is envisaged that as the invention is used, the feedback from actual gene hunting studies will allow modifications of the manner in which the input data is used. If a theoretical patient population is produced whose size, relative to the test population, is less than the relative size of the actual patient population, in other words, namely, that the disease is more prevalent than expected, obviously, it would be necessary to alter the initial population or to alter the manner in which the population was generated by, for example, introducing the mutation in at least one additional locus so as to get a new theoretical patient population. Similarly, when the test population provided a theoretical patient population whose size relative to the test population is greater than the relative size of the actual patient population, then the size of the initial population can be increased and a new test population produced. It will be appreciated that there are many variations and manners in which the initial population, the insertion of the mutation and test loci, the number of generations over which the population can be generated, and so on, are all variables that can be changed and indeed, as more experience from practical gene hunting studies is provided, the invention will be adapted having regard to these results.

Essentially, the invention has two advantages. The first is that it can theoretically check a gene hunting study and find out whether it could possibly ever work. If a proposed gene hunting study could be shown to be theoretically impossible to lead to a satisfactory result, then the proposed gene hunting study could be abandoned before it was started. Alternatively, the method of the present invention will allow the gene hunting study to be defined and designed in the correct manner.

It is envisaged that the gene hunting study according to the present invention and indeed the practical aspects of an actual gene hunting study may not be carried out where the invention is carried out. For example, the invention could be contained on a server in a remote location and a research might simply input data to the server, which server would then either return to the researcher a test population and thus a patient population to allow the researcher carry out his or her theoretical gene hunting studies or alternatively, the server could provide the results of the theoretically gene hunting study to the researcher or investigator. It is estimated that this could all be done over the internet or other communications networks.

It will be appreciated that this invention can be carried out by a server or computer which will be programmed to provide various means for carrying out the invention such as means to generate a test population from an initial population of a certain size in which the test population is expressed in terms of its allelic data. There can also be means to input a mutation at at least one locus during generation of the test population or indeed at many loci. Similarly, there can be provided means to extract a theoretical patient population from the test population having regard to its allelic data. Further, there can be various means to carry out gene hunting studies on at least part of the theoretical patient population and to carry out a study testing methodology. It will also be noted from the comments above that means can be provided to carry out all of the methods of the present invention.

It will be appreciated that various aspects of the invention may be embodied on a computer that is running a program or program segments originating from a computer readable or usable medium, such medium including but not limited to magnetic storage media (e.g. ROMs, floppy disks, hard disks, etc.), optically readable media (e.g. CD-ROMs, DVDs, etc.) and carrier waves (e.g., transmissions over the internet). A functional program, code and code segments, used to implement the present invention can be derived by a skilled computer programmer from the description of the invention contained herein.

It will be appreciated therefore that a computerised program may be provided providing program instructions which, when loaded into a computer, will constitute the means in accordance with the invention and that this computer program may be embodied on a record medium, a computer memory, a read only memory or carried on an electrical carrier signal.

In the specification the terms "comprise, comprises, comprised and comprising" or any variation thereof and the terms "include, includes, included and including" or any variation thereof are considered to be totally interchangeable and they should all be afforded the widest possible interpretation.

The invention is not limited to the embodiments hereinbefore described but may be varied in both construction and detail within the scope of the appended claims.

## Claims

1. A method of defining a gene hunting study to determine certain genetic data comprising:-
specifying the loci of reference markers;
specifying the locus of at least one test locus;
obtaining the recombination fraction between all the loci;
defining an initial population including at least its size and allelic data for the generation of a test population of individuals identified by their alleles;
generating a test population from the initial population by causing the initial population to grow into a test population;
inputting a mutation at the or each test locus during the generation of the test population; and
selecting the required genetic data from the test data having regard to the mutation and reference markers to provide a theoretical patient population having the mutation to allow an initial assessment of the gene hunting study.

2. A method as claimed in claim 1, comprising:-
using the theoretical patient population to carry out a gene hunting study; and
using the results of this gene hunting study to specify an actual gene hunting study.

3. A method as claimed in claim 1 or 2 in which generating the test population comprises:
selecting parents from the initial population in accordance with predetermined parental choice rules;
determining the number of offspring for each set of parents in accordance with predetermined birth rate rules;
assigning a gender to each offspring in accordance with predetermined gender rules;
allocating genes to each offspring allowing for random recombinations in accordance with specified recombination parameters; and
retiring members of the population in accordance with predetermined mortality rates.

4. A method as claimed in any preceding claim in which the recombination fraction assigned to the test locus is obtained by taking a portion of the recombination fraction of the two adjacent markers.

5. A method as claimed in claim 4 in which the recombination fraction assigned is half the recombination fraction of the two adjacent markers.

6. A method as claimed in claim 4 in which when there is more than one test locus between adjacent markers the sum of the recombination fraction is a proportion of the recombination fraction between the markers.

7. A method as claimed in any preceding claim, in which the test population is chosen after many generations of evolution.

8. A method as claimed in claim 7, in which, at predetermined numbers of generations, a correlation between reference alleles and other alleles is determined and when the correlation is determined to be satisfactory, the test population is chosen to be that size for extraction of a patient size for the subsequent study.

9. A method as claimed in any preceding claim, in which the size of the initial population is varied and test populations are determined for different sizes of initial population.

10. A method as claimed in any preceding claim in which the mutation is introduced in the initial population.

11. A method as claimed in any of claims 1 to 9 in which the mutation is introduced many generations beyond the initial population.

12. A method as claimed in claim 10 or 11 in which the mutation is introduced at a number of loci.

13. A method as claimed in any preceding claim in which the size of the theoretical population is varied to determine the size of a subsequent real patient population for the actual gene hunting study.

14. A method as claimed in any preceding claim in which when the test population provides a theoretical patient population whose size relative to the test population is less than the relative size of the actual patient population the mutation is introduced at at least one additional locus and a new theoretical patient population produced.

15. A method as claimed in any of claims 1 to 13 in which when the test population provides a theoretical patient population whose size relative to the test population is greater than the relative size of the actual patient population the size of the initial population is increased and a new test population produced.

16. A method as claimed in any preceding claim in which a plurality of theoretical patient populations of different allelic data are chosen for the one gene hunting study and a theoretical gene hunting study is carried out on each theoretical patient population to provide one or more real patient populations for the subsequent actual study.

17. A method as claimed in any preceding claim in which the efficacy of the gene hunting study in the theoretical patient population is assessed by using a gene hunting study testing methodology.

18. A method as claimed in claim 17 in which more than one gene testing methodology is used.

19. A method as claimed in any preceding claim in which more than one gene hunting methodology is used.

20. A method as claimed in any preceding claim in which an actual gene hunting study including the specification of the loci of reference alleles and size of patient population is defined when a gene study on a theoretical patient population provides an apparently successful study.

21. A method as claimed in any preceding claim comprising:
providing at least some of the data necessary to carry out the method of any preceding claim;
sending the data to a third party;
receiving the results of the method carried out by the third party; and
using the results to carry out a subsequent gene hunting study.

22. A theoretical gene hunting study produced by the method of any preceding claim.

23. An actual gene hunting study which was carried out after being defined by the method of any of claims 1 to 21.

24. A system for control of a gene hunting study to determine certain genetic data including a server comprising:
means to generate a test population from an initial population of a certain size in which the test population is expressed in terms of its allelic data;
means to input a mutation at least one locus during generation of the test population; and
means to extract a theoretical patient population from the test population having regard to its allelic data.

25. A system as claimed in claim 24 comprising means to carry out a gene hunting study on at least part of the theoretical patient population.

26. A system as claimed in claim 25 comprising means to carry out a study testing methodology.

27. A computer program comprising program instructions for causing a computer to perform the method of any of claims 1 to 21.

28. A computer program comprising program instructions which when loaded into a computer comprise the means of any of claims 24 to 26.

29. A computer program as claimed in claim 27 or 28 embodied on a record medium.

30. A computer program as claimed in claim 27 or 28 stored in a computer memory.

31. A computer program as claimed in claim 27 or 28 embodied in a read only memory.

32. A computer program as claimed in claim 27 or 28 carried on an electrical signal carrier.
